**Europäisches Patentamt**

**European Patent Office**

**Office européen des brevets**

(19)

(11) Veröffentlichungsnummer : **0 449 800 A1**

(12) # EUROPÄISCHE PATENTANMELDUNG

(21) Anmeldenummer : **91890047.3**

(22) Anmeldetag : **18.03.91**

(51) Int. Cl.⁵ : **A61B 5/0408, A61B 5/0416**

(30) Priorität : **22.03.90 AT 677/90**

(43) Veröffentlichungstag der Anmeldung :
**02.10.91 Patentblatt 91/40**

(84) Benannte Vertragsstaaten :
**BE CH DE DK ES FR GB GR IT LI LU NL SE**

(71) Anmelder : **Bürtlmair, Hermann**
**Bauernstrasse 1**
**A-4600 Wels (AT)**
Anmelder : **Höllinger, Siegfried**
**Aspeth 31**
**A-4720 Neumarkt/H. (AT)**

(72) Erfinder : **Bürtlmair, Hermann**
**Bauernstrasse 1**
**A-4600 Wels (AT)**
Erfinder : **Höllinger, Siegfried**
**Aspeth 31**
**A-4720 Neumarkt/H. (AT)**

(74) Vertreter : **Hübscher, Heiner, Dipl.-Ing. et al**
**Spittelwiese 7**
**A-4020 Linz (AT)**

(54) **Elektrode für elektrische Messeinrichtungen und dergleichen.**

(57)     Eine Elektrode 1 für elektrische Meßeinrichtungen weist einen einstückigen aus kohlefaserhältigem Kunststoff bestehenden Sensorknopf 2 auf, der in einem plättchenförmigen Sensorträger 3 einsteckbar ist und einerseits einen in eine Kontaktgelschicht 4 eingebetteten Fussteil 2b und anderseits einen Kopfteil 2a zum Ansetzen eines Anschlußkabels 9 bildet.

    Um auch bei der Verwendung von metallfreiem, kohlefaserhältigem Kunststoff beste Leiteigenschaften zu erreichen, weist der Sensorknopf (2) ein sich axial vom Fußteil (2b) bis zum Kopfteil (2a) erstreckendes Loch (2c) auf.

EP 0 449 800 A1

Jouve, 18, rue Saint-Denis, 75001 PARIS

*FIG.1*

*FIG.2*

## ELEKTRODE FÜR ELEKTRISCHE MEßEINRICHTUNGEN OD. DGL.

Die Erfindung bezieht sich auf eine Elektrode für elektrische Meßeinrichtungen u. dgl., insbesondere medizinische Diagnostiziergeräte, mit einem einstückigen, aus kohlefaserhältigem Kunststoff bestehenden Sensorknopf, der in einen plättchenförmigen Sensorträger einsteckbar ist und einerseits einen in eine Kontaktgelschicht od. dgl. eingebetteten Fußteil und anderseits einen vorragenden Kopfteil zum Ansetzen eines Anschlußkabels bildet.

Solche Elektroden werden an der Haut eines Patienten oder an der Oberfläche eines beliebigen anderen elektrisch zu untersuchenden Gegenstandes angebracht und dienen zur Ableitung elektrischer Impulse und Aktionsströme von Organen oder anderen zu messenden Vorgängen, beispielsweise zur Erstellung eines Elektrokardiogramms oder anderer technischmedizinischer Untersuchungen. Bisher ist dabei meist der für die Übertragung der elektrischen Signale von der Haut bzw. der Oberfläche zum Anschlußkabel und damit zum Meßgerät od. dgl. verantwortliche Sonsorknopf aus einem Kunststoffkern hergestellt, der zur Gewährleistung der elektrischen Leitfähigkeit galvanisch versilbert und anschließend zum Schutz silberchloriert wird, wobei häufig anschlußseitig ein zusätzlicher metallischer Kopfteil für den Anschlußkabelansatz auf den Sensorknopf aufgedrückt ist. Diese bekannten Elektroden enthalten daher metallische Werkstoffe, die zusätzliche Untersuchungen, beispielsweise Röntgenuntersuchungen oder Untersuchungen mit Magnetresonanz-Tomographiegeräten, beeinflussen und stören würden, so daß oft gewünschte Paralleluntersuchungen unmöglich sind. Abgesehen davon ergibt sich die Notwendigkeit eines zeitaufwendigen und unangenehmen mehrfachen Aufklebens und Abnehmens der Elektroden und des damit verbundenen unwirtschaftlichen Elektrodenverbrauches, wozu noch die auf grund der galvanischen Versilberung und Silberchlorierung recht umweltgefährdende und aufwendige Herstellung der Sensorknöpfe und nicht zuletzt die Schwierigkeit der Entsorgung gebrauchter Elektroden, die wegen ihres Aufbaus als Sondermüll zu behandeln sind, kommen.

Wie die CH-PS 668 690 zeigt, wurde zur Vermeidung von metallischen Werkstoffen und zur Vereinfachung der Herstellung auch schon vorgeschlagen, Elektroden mit einstückigen, aus kohlefaserhältigem Kunststoff bestehenden Sensorknöpfen zu bestücken, doch haben sich diese als Vollkörper ausgebildeten Sonsorknöpfe wegen ihrer unbefriedigenden Leiteigenschaften, die das Erfassen feinerer elektrischer Vorgänge ausschließen, nicht bewährt.

Der Erfindung leigt daher die Aufgabe zugrunde, diese Mängel zu beseitigen und eine Elektrode der eingangs geschilderten Art zu schaffen, die sich neben ihrer Metallfreiheit und ihrer rationellen Herstellbarkeit vor allem durch ihre besonders guten elektrischen Leitfähigkeiten auszeichnet.

Die Erfindung löst diese Aufgabe dadurch, daß der Sensorknopf ein sich axial vom Fußteil bis zum Kopfteil erstrekkendes Loch aufweist. Dieses Loch hilft nicht nur, Werkstoff einzusparen, sondern gewährleistet außerdem, daß die bei der Herstellung des Knopfes, beispielsweise im Spritzgießverfahren in Strömungsrichtung mehr oder weniger ausgerichteten Kohlefasern aufgrund der Umlenkung durch den das Loch bildenden Kern ihre Orientierung verlieren und eine inhomogene Faserstruktur innerhalb des Knopfes einnehmen. Diese inhomogene Faserstruktur garantiert dann auch ohne die Verwendung von Metall die gewünschten, widerstandsarmen Leiteigenschaften des Sensorknopfes, wobei die Einstückigkeit und der sparsame Materialverbrauch die besonders rationelle Fertigung ermöglichen. Wegen der fehlenden Metallanteile des Sensorknopfmaterials bereitet auch die Entsorgung der Elektroden keine Schwierigkeiten und durch geeignete Wahl des verwendeten Kunststoffes läßt sich der Sensorknopf nicht nur gegebenenfalls umweltschonend verbrennen, sondern auch in einem entsprechenden Recycling-Verfahren wiederverwerten.

An sich können beliebige Kunststoffe verwendet werden, doch besonders günstig ist es, wenn der Sensorknopf aus 40 Vol.% kohlefasergefülltem PA 6.6 (Polyhexamethylenadipamid), vorzugsweise im Spritzgießverfahren, hergestellt ist, da dieses Polyamid leicht verwertbar und entsorgbar ist und die gewünschte Bindung für die Kohlefasern mit sich bringt. Ein Füllungsgrad von etwa 40 Vol.% ist dabei durchaus möglich.

Gehört dem Kopfteil des Sensorknopfes nach einer Weiterbildung der Erfindung ein Kontaktstück zu, das ebenfalls aus kohlefaserhältigem Kunststoff besteht und mit einem an sich bekannten Kohlefaser-Kabel verbunden ist, wird für die gesamte elektrische Signalableitung von der Elektrode an bis über die Anschlußstücke und das Anschlußkabel bis hin zum Aufnahmegerät die Verwendung metallischer Werkstücke vollkommen vermieden. Die Elektroden und die Anschlüsse können unbeschadet einer parallellaufenden Röntgen- oder Magnetuntersuchung am Körper verbleiben und es müssen nicht einmal die Anschlußkabel von den Elektroden entfernt werden. Paralleluntersuchungen sind daher unbeschränkt möglich und die Zahl der erforderlichen Elektroden wird wesentlich gemindert.

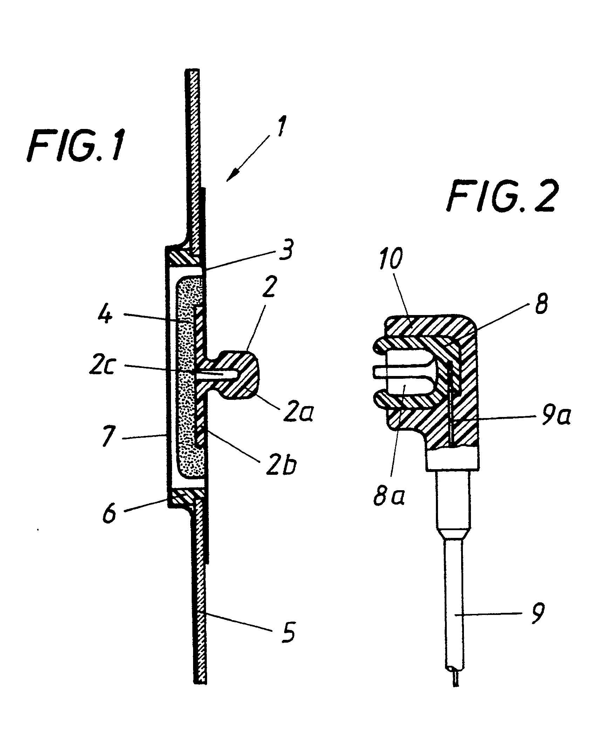

In der Zeichnung ist der Erfindungsgegenstand an Hand eines Ausführungsbeispieles rein schematisch veranschaulicht, und zwar zeigen Fig. 1 eine erfindungsgemäße Elektrode im Querschnitt und Fig.

2 ein zugehöriges Kontaktstück ebenfalls im Querschnitt.

Eine Elektrode 1 weist einen Sensorknopf 2 auf, der in einen plättchenförmigen Sensorträger 3 eingesteckt ist. Der Sensorknopf 2 bildet einerseits einen vorragenden Kopfteil 2a zum Ansetzen eines Anschlußkabels und anderseits einen Fußteil 2b, der in eine Kontaktgelschicht, beispielsweise ein mit Kontaktgel getränkter Schaumstoffpolster 4, eingebettet ist. Eine die Kontaktgelschicht umgebende kleberbeschichtete Haftfolie 5 ist am Sensorträger 3 festgeklebt und dient ihrerseits zur Befestigung der Elektrode 1 am zu untersuchenden Objekt. Zum Schutz der Kontaktgelschicht und des Sensorknopfes gibt es einen Distanzring 6 und eine den Distanzring 6 und die Haftfolie 5 abdeckende Schutzfolie 7. Vor Gebrauch der Elektrode 1 wird diese Schutzfolie 7 mit dem Distanzring 6 abgezogen und die Haftfolie 5 kontaktgelseitig auf der Haut des Patienten od. dgl. aufgeklebt. Statt Schutzfolie und Distanzring kann selbstverständlich auch eine napfförmig tiefgezogene Abdeckung od. dgl. als Schutz Verwendung finden.

Der Sensorknopf 2 ist zur Vermeidung metallischer Teile in der Elektrode 1 aus einem kohlefaserhältigen Kunststoff, vorzugsweise PA 6.6 im Spritzgießverfahren hergestellt, wobei ein axial sich vom Fußteil 2b bis zum Kopfteil 2a erstreckendes Loch 2c vorgesehen ist, um für eine die elektrischen Leitfähigkeiten des Materials verbessernde inhomogene Faserorientierung im Knopf 2 beim Spritzgießen zu sorgen.

In Fig. 2 ist ein dem Sensorknopf 2 zugeordnetes Kontaktstück 8 veranschaulicht, das zum Anstecken des Anschlußkabels 9 dient. Dieses Kontaktstück 8 ist ebenfalls aus kohlefaserhältigem Kunststoff hergestellt und bildet eine federnde Buchse 8a od. dgl., die zum Aufsetzen auf den Kopfteil 2a des Sensorknopfes 2 geeignet ist, wobei die spezielle Form und Ausgestaltung dieser Buchse ohne Bedeutung bleibt. Zum Schutz und zur besseren Handhabung ist das Kontaktstück 8 in eine weiche Kunststoffassung 10 eingebettet, die die federnde Wirkung der Buchse verstärkt und die Verbindung zwischen Kontaktstück 8 und Anschlußkabel 9 vor Beschädigungen schützt. Das Anschlußkabel 9 besteht ebenfalls aus einem Kohlefaserkabel, wobei die Kohlefasern 9a in das Kontaktstück 8 eingegossen sind, um eine möglichst widerstandsfreie Verbindung zwischen Kontaktstück 8 und Kabel 9 herzustellen.

Durch die Verwendung von kohlefaserhältigem Kunststoff für die Herstellung des Sensorknopfes 2 und auch des Kontaktstückes 8 bzw. des Anschlußkabels 9 ist eine Ableitung der elektrischen Signale vom Patienten oder einem anderen zu untersuchenden Gegenstand ohne Benützung von Metallen möglich, was den Einsatz der Elektroden und Anschlußkabel ohne störenden Einfluß auf etwaige Paralleluntersuchungen erlaubt und entscheidende Vorteile hinsichtlich Handhabung, Herstellung und Entsorgung mit sich bringt.

**Patentansprüche**

1. Elektrode (1) für elektrische Meßeinrichtungen u. dgl., insbesondere medizinische Diagnostiziergeräte, mit einem einstückigen, aus kohlefaserhältigem Kunststoff bestehenden Sensorknopf (2), der in einen plättchenförmigen Sensorträger (3) einsteckbar ist und einerseits einen in eine Kontaktgelschicht od. dgl. (4) eingebetteten Fußteil (2b) und anderseits einen vorragenden Kopfteil (2a) zum Ansetzen eines Anschlußkabels (9) bildet, dadurch gekennzeichnet, daß der Sensorknopf (2) ein sich axial vom Fußteil (2b) bis zum Kopfteil (2a) erstreckendes Loch (2c) aufweist.

2. Elektrode nach Anspruch 1, dadurch gekennzeichnet, daß der Sensorknopf (2) aus 40 Vol.% kohlefasergefülltem PA 6.6 (Polyhexamethylenadipamid), vzw. im Spritzgießverfahren, hergestellt ist.

3. Elektrode nach Anspruch 1 oder 2, dadurch gekennzeichnet, daß dem Kopfteil (2a) des Sensorknopfes (2) ein Kontaktstück (8) zugehört, das ebenfalls aus kohlefaserhältigem Kunststoff besteht und mit einem an sich bekannten Kohlefaser-Kabel (9) verbunden ist.

**FIG.1**

3

2

4

2c

2a

7

2b

6

5

**FIG.2**

10

8

9a

8a

9

Europäisches
Patentamt

# EUROPÄISCHER RECHERCHENBERICHT

Nummer der Anmeldung

EP     91 89 0047

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | CH-A-668690 (REMATRA)<br>* Seite 2, Zeilen 10 - 39; Figuren *<br>--- | 1-3 | A61B5/0408<br>A61B5/0416 |
| A | US-A-4640289 (L.W. CRAIGHEAD)<br>* Spalte 4, Zeilen 25 - 46; Figur 2 *<br>--- | 1 | |
| A | US-A-3976055 (R.P. MONTER ET AL.)<br>* Spalte 3, Zeilen 3 - 15; Figuren *<br>* Spalte 7, Zeilen 29 - 40 *<br>* Spalte 13, Zeile 1 - Spalte 14, Zeile 34 *<br>--- | 1, 2 | |
| A | EP-A-210020 (BAXTER TRAVENOL LABS., INC.)<br>* Seite 1, Zeilen 1 - 13; Figuren *<br>* Seite 6, Zeile 24 - Seite 8, Zeile 7 *<br>* Seite 9, Zeile 23 - Seite 10, Zeile 27 *<br>--- | 1, 3 | |
| A | PATENT ABSTRACTS OF JAPAN<br>vol. 12, no. 340 (M-740)(3187) 13 September 1988,<br>& JP-A-63 099930 (KANEBO LTD.) 02 Mai 1988,<br>* das ganze Dokument *<br>--- | 2 | |
| A | EP-A-188302 (REMATRA)<br>----- | | |

RECHERCHIERTE
SACHGEBIETE (Int. Cl.5 )

A61B

Der vorliegende Recherchenbericht wurde für alle Patentansprüche erstellt

| Recherchenort | Abschlußdatum der Recherche | Prüfer |
|---|---|---|
| DEN HAAG | 21 MAI 1991 | RIEB K.D. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument

....................................................................

& : Mitglied der gleichen Patentfamilie, übereinstimmendes
   Dokument

EPO FORM 1503 03.82 (P0403)